# EUROPEAN PATENT SPECIFICATION

(11) **EP 1 337 533 B1**
(45) Date of publication and mention of the grant of the patent: **17.05.2006**
(21) Application number: 01978740.7
(22) Date of filing: 26.10.2001
(51) Int. Cl.: C07D 471/04, C07D 241/00, C07D 221/00

(54) **RESOLUTION OF TRANS-7-(HYDROXY-METHYL)OCTA-HYDRO-2H-PYRIDO-1,2A)PYRAZINE**
RACEMATSPALTUNG VON TRANS 7-(HYDROXYMETHYL)OCTAHYDRO-2H-PYRIDO¬1,2-A|PYRAZIN
RESOLUTION DE TRANS-7-(HYDROXY-METHYL)OCTA-HYDRO-2H-PYRIDO-1,2A)PYRAZINE

(30) Priority: 29.11.2000 US 250074 P
(43) Date of publication of application: 27.08.2003
(73) Proprietor: Pfizer Products Inc., Groton, Connecticut 06340 (US)
(72) Inventor: HARMS, Arthur, Eugene, Niskayuna, NY 12309 (US)
(74) Representative: Rutt, Jason Edward
(86) International application number: PCT/IB2001/002018
(87) International publication number: WO 2002/044177

(56) References cited:
- WO-A-92/13858

## Description

### Background of the Invention

The present invention relates to a process for the optical resolution of a key intermediate for preparing pharmacologically active 2,7-substituted octahydro-1H-pyrido[1,2-a]pyrazine derivatives, such as (7S,trans)-2-(2-pyrimldinyl)-7-(hydroxymethyl)octahydro-2H-pyrido(1,2-a)pyrazine, which are disclosed in U.S. Patent No. 5,852,031, the contents of which are hereby incorporated by reference. These pyrazine compounds are ligands with specificity for dopamine receptor subtypes, especially the dopamine D4 receptor, within the animal body, and are therefore useful in the treatment of disorders of the dopamine system. The process of the present invention involves resolution of trans-7-(hydroxymethyl)octahydro-2H-pyrido(1,2-a)pyrazine using D-(-) or L-(+)naproxen.

Previously, the desirable optically resolved pyrazine compounds were obtained by later-stage resolution using D-(-) or L-(+)-tartaric acid, as disclosed in European Patent No. 569387. The present method has the advantage of minimizing material losses incurred by conducting resolutions after a multistep synthetic sequence, and results in a more efficient, higher-yielding process for preparing the (7S,trans)-2-(2-pyrimidinyl)-7-(hydroxymethyl)octahydro-2H-pyrido(1,2-a)pyrazines.

### Summary of the Invention

The present invention relates to a process for separating a racemic mixture, or an optically enriched mixture, of trans-7-(hydroxymethyl) octahydro-2H-pyrido(1,2-a)pyrazine containing a first enantiomer having the formula: 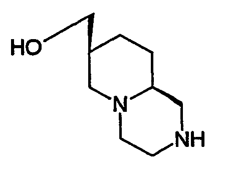 and a second enantiomer having the formula: 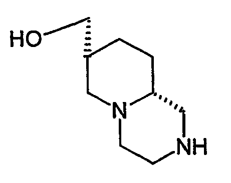 the process comprising:
reacting the racemic mixture, or the optically enriched mixture, with (+)-naproxen or (-)-naproxen to form, respectively, a diastereomeric niixture of the (+)- or (-)-naproxen salts of each of the enantiomers; separating each of the diastereomeric (+)- or (-)-naproxen salts; and if desired, converting the respective naproxen salt of each enantiomer to the free base thereof.

The present invention further provides a salt of a compound with a substance selected from the group consisting of (+)-naproxen and (-)-naproxen, said compound having the formula

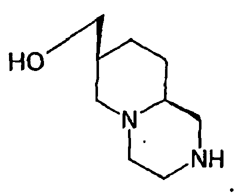

The present invention also provides a salt of a compound with a substance selected from the group consisting of (+)-naproxen and (-)-naproxen, said compound having the formula

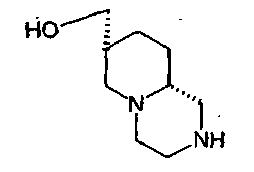

### Brief Description of Drawings

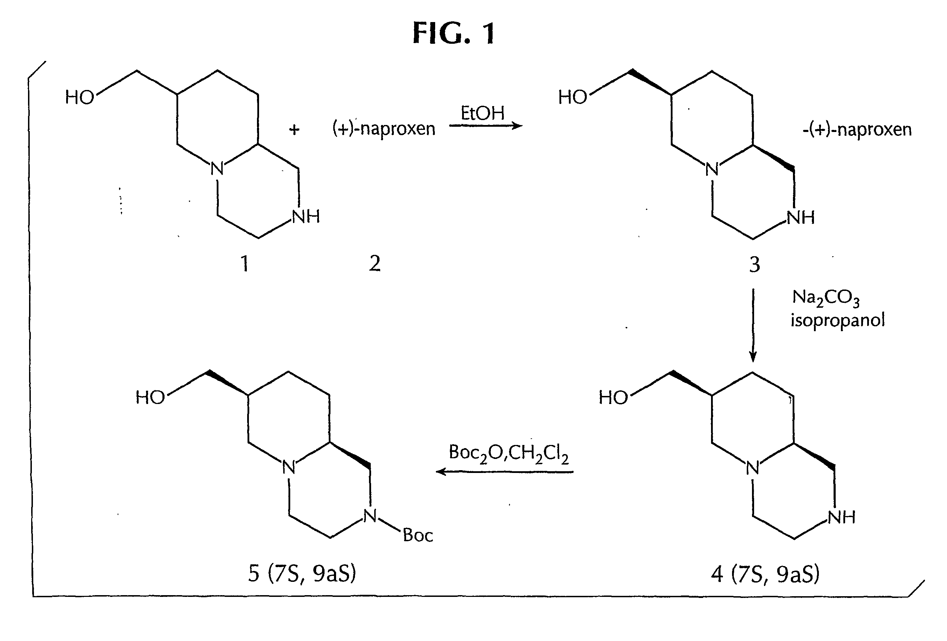

Figure 1 shows a synthetic pathway for preparing key intermediates in the manufacture of biologically active 2,7-substituted octahydro-1H-pyrido[1,2-a]pyrazine derivatives.

### Detailed Description of the Invention

As illustrated in the accompanying Figure, the present invention provides a process for resolution of a racemic mixture, or an optically enriched mixture, of pyrazines which comprises forming a (+)- or (-)-naproxen salt (2) of the pyrazines (1) and thereafter, in a separating step, converting the respective (+)- or (-)-naproxen salt to the free-base of the associated enantiomer (3). Preferably, in one implementation of the process of the invention, the racemic mixture, or the optically enriched mixture, is reacted with (+)-naproxen. The amount of (+)-naproxen added in the reaction is from about 0.5 equivalent to about 1.0 equivalent, and is preferably about 0.5 equivalent. In the process, the separating step comprises isolating an insoluble (+)-naproxen salt from a soluble (+)-naproxen salt, and the insoluble (+)-naproxen salt is a salt of the first enantiomer.

Alternatively, the process of the present invention may be performed wherein the racemic mixture, or the optically enriched mixture, of pyrazines is reacted with (-)-naproxen, In such case, the separating step comprises isolating an insoluble (-)-naproxen salt from a soluble (-)-naproxen salt, and the insoluble (-)-naproxen salt is a salt of the second enantiomer. In one embodiment, the process comprises a reacting step which comprises contacting the racemic mixture, or the optically enriched mixture, with (+)-naproxen to form a precipitate in a solution, wherein the precipitate is the (+)-naproxen salt of the first enantiomer; and the process further comprises separating the precipitate from the solution; and further comprising converting the (+)-naproxen salt of the first enantiomer to the free-base thereof.

The reacting step optionally comprises contacting the racemic mixture, or the optically enriched mixture, with (-)-naproxen to form a precipitate in a solution, the solution comprising the (-)-naproxen salt of the first enantiomer dissolved therein; the separating step comprises separating the precipitate from the solution and evaporating the solution so as to provide the salt of the first enantiomer; and further comprising converting the (-)-naproxen salt of the first enantiomer to the free-base thereof.

Accordingly, in the process of the present invention, trans-7-(hydroxymethyl)octahydro-2H-pyrido(1,2-a)pyrazine is resolved using (+)-naproxen or (-)-naproxen. The pyrazine is reacted with either (+)-naproxen or (-)-naproxen in an inert polar solvent. Suitable solvents include methanol, ethanol, isopropanol, ethyl acetate, diethyl ether and tetrahydrofuran, or mixtures thereof. Ethanol is a preferred solvent. The temperature of the reaction is not critical. Generally, the reaction mixture will be heated to a temperature sufficient to dissolve the starting material (i.e., about 30 to about 50°C, preferably about 40°C) and then allowed to cool. Upon cooling, one of the diastereoisomeric (+)-naproxen or (-)-naproxen salts precipitates. When the (+)-naproxen salt is formed, the 7R enantiomer remains in solution and the 7S enantiomer precipitates. When the (-)-naproxen salt is formed, the 7S enantiomer remains in solution and the 7R enantiomer precipitates. If the desired enantiomer remains in solution, it is recovered by evaporating the liquid.

In order to convert the resulting isolated salt to the free base, any suitable method known in the art may be applied. Preferably, the salt is dissolved in water and the pH is raised to between about 10 to about 14, preferably about pH 12, using a base, such as sodium hydroxide, sodium bicarbonate, sodium carbonate, potassium hydroxide, potassium bicarbonate, potassium carbonate, ammonium hydroxide, and the like. The free base pyrazine is extracted from the aqueous layer using an inert non-polar solvent, such as isopropyl ether, diethyl ether, 1,1,1-trichloroethane,' or methylene chloride, preferably the latter.

Pharmacologically useful pyrazine compounds may be prepared using the optically resolved intermediates as described herein. Among the uses are the amelioration of the symptoms of anxiety and other psychiatric conditions in a human subject. Methods for further synthetic elaboration of the optically resolved pyrazines to the pharmaceutical targets are disclosed in U.S. Patent No. 5,852,031. The pyrazines prepared thereby are administered in accordance with methods known in the art in an effective amount of about 2 to about 200 mg/day, in single or divided daily doses. In particular cases, dosages outside that range are prescribed at the discretion of the attending physician. The preferred route of administration is generally oral, but parenteral administration (e.g., intramuscular, intravenous, intradermal) will be preferred in special cases, e.g. where oral absorption is impaired as by disease, or the patient is unable to swallow. These compounds are generally administered in the form of pharmaceutical compositions comprising a pharmaceutically acceptable vehicle or diluent. Such are generally formulated in a conventional manner utilizing solid or liquid vehicles or diluents as appropriate to the mode of desired administration: for oral administration, in the form of tablets, hard or soft gelatin capsules, suspensions, granules, powders and the like; and, for parenteral administration, in the form of injectable solutions or suspensions, and the like.

The present invention is illustrated, but not limited, by the following examples.

### EXAMPLE 1

### Diastereomeric Salt 2 of trans-7-(hydroxymeth-yl)octahydro-2H-pyr-ido(1,2-a)pyrazine

Five grams (29.2 mmol) of racemic trans-7-(hydroxymethyl)octa-hydro-2H-pyrido(1,2-a)pyrazine (*rac*-**1**) were dissolved in 25 ml of ethanol. (+)-Naproxen (3.2 g, 13.9 mmol) was added. Upon stirring, a solid began to precipitate. After stirring at room temperature for 48 hours the reaction was filtered and the solids dried. The diastereomeric salt (2; 3.1 grams) of trans-7-(hydroxymethyl)octahydro-2H-pyrido(1,2-a)pyrazine was thereby obtained (56% yield based on one enantiomer). [α]_{D} = -13.4° (c=1.11, MeOH).

### EXAMPLE 2

### (7S,trans)-7-(Hydroxymethyl)octahydro-2H-pyrido(1,2-a)pyrazine (7S-3)

A solution of 3.0 g of diastereomeric salt (**2**) of trans-7-(hydroxymeth-yl)octahydro-2H-pyrido(1,2-a)pyrazine (7.49 mmol) in isopropanol was heated to reflux. 4.0 g of Na₂CO₃ (37.4 mmol) was added and the mixture was refluxed for 17 hr. The reaction was cooled to room temperature and filtered. The solids were washed with isopropanol and the filtrate was concentrated to dryness. The solids thus obtained were treated with hot toluene and filtered to remove insoluble salts. The filtrate was cooled and filtered to yield 0.9 g of a white solid (7S-3; 70%). [α]_{D} = -24.8° (c=0.895, MeOH). An authentic sample of (7S,trans)-7-(hydroxymethyl)octahydro-2H-pyrido(1,2-a)pyrazine showed [α]_{D} = -27.0° (c=0.895, MeOH) indicating that the material produced here was approximately 92% ee.

## Claims

1. A process for separating a racemic mixture, or an optically enriched mixture, of a first enantiomer of the formula 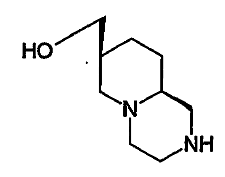 and a second enantiomer of the formula 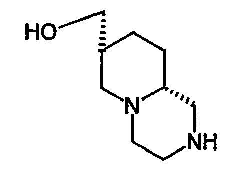 comprising:
reacting the racemic mixture, or the optically enriched mixture, with (+)-naproxen or (-)-naproxen to form respectively a diastereomeric mixture of the (+)- or (-) naproxen salts of each of the enantiomers; and separating each of the diastereomeric (+)- or (-)-naproxen salts.

2. The process of Claim 1, further comprising, after said separating step, converting at least one of the (+)- or (-)-naproxen salts to the free-base of the respective separated enantiomers.

3. The process of Claim 1, wherein said racemic mixture, or the optically enriched mixture, is reacted with (+)-naproxen.

4. The process of Claim 3, wherein said separating step comprises isolating an insoluble (+)-naproxen salt from a soluble (+)-naproxen salt.

5. The process of Claim 4, wherein said insoluble (+)-naproxen salt is a salt of the first enantiomer.

6. The process of Claim 1, wherein the racemic mixture, or the optically enriched mixture, is reacted with (-)-naproxen.

7. The process of Claim 6, wherein said separating step comprises isolating an insoluble (-)-naproxen salt from a soluble (-)-naproxen salt.

8. The process of Claim 7, wherein said insoluble (-)-naproxen salt is a salt of the second enantiomer.

9. The process of Claim 1, wherein said reacting step comprises contacting the racemic mixture, or the optically enriched mixture, with (+)-naproxen to form a precipitate in a solution, said precipitate being the (+)-naproxen salt of the first enantiomer; said separating step comprises separating the precipitate from the solution; and further comprising converting the (+)-naproxen salt of the first enantiomer to the free-base thereof.

10. The process of Claim 1, wherein said reacting step comprises contacting the racemic mixture with (-)-naproxen to form a precipitate in a solution, the solution comprising the (-)-naproxen salt of the first enantiomer dissolved therein; said separating step comprises separating the precipitate from the solution and evaporating the solution so as to provide the salt of the first enantiomer, and further comprising converting the (-)-naproxen salt of the first enantiomer to the free-base thereof.

11. A salt of a compound with a substance selected from the group consisting of (+)-naproxen and (-)-naproxen, said compound having the formula

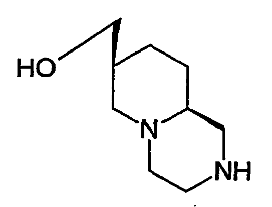

12. A salt of a compound with a substance selected from the group consisting of (+)-naproxen and (-)-naproxen, said compound having the formula

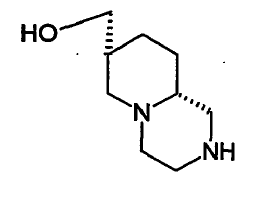

## Patentansprüche

1. Verfahren zur Trennung eines racemischen Gemisches oder eines optisch angereicherten Gemisches eines ersten Enantiomeren der Formel 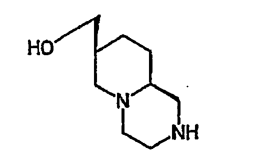 und eines zweiten Enantiomeren der Formel 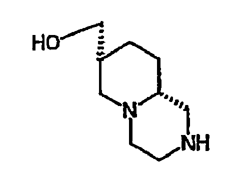 umfassend:
die Umsetzung des racemischen Gemisches oder des optisch angereicherten Gemisches mit (+)-Naproxen oder (-)-Naproxen, um ein diastereomeres Gemisch der (+)- bzw. (-)-Naproxensalze jedes der Enantiomeren zu bilden; und die Trennung jedes der diastereomeren (+)- oder (-)-Naproxensalze.

2. Verfahren nach Anspruch 1, weiterhin umfassend, nach der genannten Trennstufe, die Umwandlung mindestens eines der (+)- oder (-)-Naproxensalze in die freie Base der jeweiligen abgetrennten Enantiomeren.

3. Verfahren nach Anspruch 1, wobei das genannte racemische Gemisch oder das optisch angereicherte Gemisch mit (+)-Naproxen umgesetzt wird.

4. Verfahren nach Anspruch 3, wobei die genannte Trennstufe die Abtrennung eines unlöslichen (+)-Naproxensalzes von einem löslichen (+)-Naproxensalz umfasst.

5. Verfahren nach Anspruch 4, wobei das genannte unlösliche (+)-Naproxensalz ein Salz des ersten Enantiomeren ist.

6. Verfahren nach Anspruch 1, wobei das racemische Gemisch oder das optisch angereicherte Gemisch mit (-)-Naproxen umgesetzt wird.

7. Verfahren nach Anspruch 6, wobei die genannte Trennstufe die Abtrennung eines unlöslichen (-)-Naproxensalzes von einem löslichen (-)-Naproxensalz umfasst.

8. Verfahren nach Anspruch 7, wobei das genannte unlösliche (-)-Naproxensalz ein Salz des zweiten Enantiomeren ist.

9. Verfahren nach Anspruch 1, wobei die genannte Stufe der Umsetzung die Kontaktierung des racemischen Gemisches oder des optisch angereicherten Gemisches mit (+)-Naproxen zur Bildung eines Niederschlags in einer Lösung umfasst, wobei der genannte Niederschlag aus dem (+)-Naproxensalz des ersten Enantiomeren besteht; und wobei die genannte Trennstufe die Trennung des Niederschlags von der Lösung umfasst; und wobei das Verfahren weiterhin die Umwandlung des (+)-Naproxensalzes des ersten Enantiomeren in die freie Base davon umfasst.

10. Verfahren nach Anspruch 1, wobei die genannte Stufe der Umsetzung die Kontaktierung des racemischen Gemisches mit (-)-Naproxen zur Bildung eines Niederschlags in einer Lösung umfasst, wobei die Lösung darin gelöst das (-)-Naproxensalz des ersten Enantiomeren umfasst; wobei die genannte Trennstufe die Trennung des Niederschlags von der Lösung und das Eindampfen der Lösung, so dass das Salz des ersten Enantiomeren erhalten wird, umfasst; und wobei das Verfahren weiterhin die Umwandlung des (-)-Naproxensalzes des ersten Enantiomeren in die freie Base davon umfasst.

11. Salz einer Verbindung mit einer Substanz, ausgewählt aus der Gruppe, bestehend aus (+)-Naproxen und (-)-Naproxen, wobei die genannte Verbindung die Formel 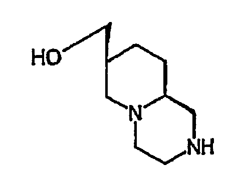 hat.

12. Salz einer Verbindung mit einer Substanz, ausgewählt aus der Gruppe, bestehend aus (+)-Naproxen und (-)-Naproxen, wobei die genannte Verbindung die Formel 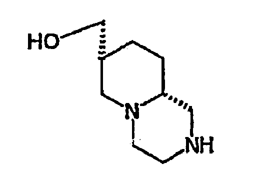 hat.

## Revendications

1. Procédé de séparation d'un mélange racémique, ou d'un mélange enrichi optiquement, comprenant un premier énantiomère de formule : 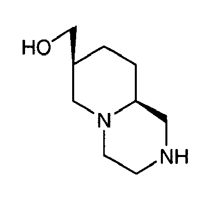 et un second énantiomère de formule : 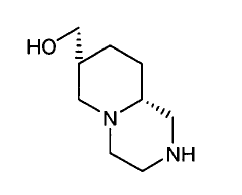 ledit procédé comprenant :
la réaction du mélange racémique, ou du mélange enrichi optiquement, avec du (+)-naproxène ou du (-)-naproxène pour former, respectivement, un mélange diastéréomère de sels de (+)- ou (-)-naproxène de chacun des énantiomères ; et
la séparation de chacun des sels diastéréomères de (+)- ou (-)-naproxène.

2. Procédé selon la revendication 1, comprenant en outre, après ladite étape de séparation, la transformation de l'un au moins des sels de (+)- ou (-)-naproxène en la base libre des énantiomères séparés respectifs.

3. Procédé selon la revendication 1, dans lequel ledit mélange racémique, ou le mélange enrichi optiquement, est mis à réagir avec du (+)-naproxène.

4. Procédé selon la revendication 3, dans lequel ladite étape de séparation comprend l'isolement d'un sel de (+)-naproxène insoluble à partir d'un sel de (+)-naproxène soluble.

5. Procédé selon la revendication 4, dans lequel ledit sel de (+)-naproxène insoluble est un sel du premier énantiomère.

6. Procédé selon la revendication 1, dans lequel le mélange racémique, ou le mélange enrichi optiquement, est mis à réagir avec du (-)-naproxène.

7. Procédé selon la revendication 6, dans lequel ladite étape de séparation comprend l'isolement d'un sel de (-)-naproxène insoluble à partir d'un sel de (-)-naproxène soluble.

8. Procédé selon la revendication 7, dans lequel ledit sel de (-)-naproxène insoluble est un sel du second énantiomère.

9. Procédé selon la revendication 1, dans lequel ladite étape de réaction comprend la mise en contact du mélange racémique, ou du mélange enrichi optiquement, avec du (+)-naproxène pour former un précipité dans une solution, ledit précipité étant le sel de (+)-naproxène du premier énantiomère ; ladite étape de séparation comprenant la séparation du précipité depuis la solution ; et comprenant, en outre, la transformation du sel de (+)-naproxène du premier énantiomère en la base libre de celui-ci.

10. Procédé selon la revendication 1, dans lequel ladite étape de réaction comprend la mise en contact du mélange racémique avec du (-)-naproxène pour former un précipité dans une solution, la solution comprenant le sel de (-)-naproxène du premier énantiomère sous forme dissoute ; ladite étape de séparation comprenant la séparation du précipité depuis la solution et l'évaporation de la solution pour obtenir le sel du premier énantiomère ; et comprenant, en outre, la transformation du sel de (-)-naproxène du premier énantiomère en la base libre de celui-ci.

11. Sel d'un composé avec une substance sélectionnée dans le groupe consistant en le (+)-naproxène et le (-)-naproxène, ledit composé étant de formule :

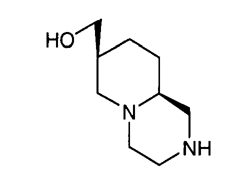

12. Sel d'un composé avec une substance sélectionnée dans le groupe consistant en le (+)-naproxène et le (-)-naproxène, ledit composé étant de formule :
